# EUROPÄISCHE PATENTANMELDUNG

(11) **EP 2 143 425 A1**
(43) Veröffentlichungstag der Anmeldung: **13.01.2010**
(21) Anmeldenummer: 08012558.6
(22) Anmeldetag: 11.07.2008
(51) Int. Cl.: A61K 9/20, A61K 9/16, A61K 31/165

(54) **Direktverpresste Aliskiren-Tabletten**

(71) Anmelder: Ratiopharm GmbH, 89079 Ulm (DE)
(72) Erfinder: Muskulus, Frank, 82194 Gröbenzell (DE); Pätz, Jana, 86424 Dinkelscherben (DE)
(74) Vertreter: Kalhammer, Georg

(57) **Zusammenfassung**

Die Erfindung betrifft pharmazeutische Zusammensetzungen, welche den Wirkstoff Aliskiren enthalten und zur Herstellung von Tabletten durch Direktverpressung geeignet sind, sodass auf eine vorhergehende Naßgranulierung verzichtet werden kann. Ferner betrifft die Erfindung Tabletten, welche durch Direktverpressung dieser pharmazeutischen Zusammensetzungen erhältlich sind sowie ein Verfahren zur Herstellung dieser Tabletten. Weiterhin betrifft die Erfindung die Verwendung der neuen pharmazeutischen Zusammensetzungen und Tabletten zur Behandlung von Hypertonie und damit assoziierten Erkrankungen.

## Beschreibung

Die Erfindung betrifft pharmazeutische Zusammensetzungen, welche den Wirkstoff Aliskiren enthalten und zur Direktverpressung bzw. Trockenverarbeitung geeignet sind, sodass auf eine vorhergehende Naßgranulierung des Wirkstoffes mit Hilfsstoffen verzichtet werden kann. Ferner betrifft die Erfindung Tabletten, welche durch Direktverpressung dieser pharmazeutischen Zusammensetzungen erhältlich sind, sowie ein Verfahren zur Herstellung dieser Tabletten. Weiterhin betrifft die Erfindung die Verwendung der neuen pharmazeutischen Zusammensetzungen und Tabletten zur Behandlung von Hypertonie und damit assoziierten Erkrankungen.

Aliskiren (IUPAC-Name: (2*S*,4*S*,5*S*,7*S*)-5-amino-*N*-(2-carbamoyl-2-methyl-propyl)-4-hydroxy -7-{[4-methoxy-3-(3-methoxypropoxy)phenyl]methyl}-8-methyl-2-propan-2-yl-nonanamid) ist ein direkter Renin-Inhibitor, der zur Senkung von erhöhtem Blutdruck eingesetzt wird. Aliskiren hat die folgende chemische Struktur:

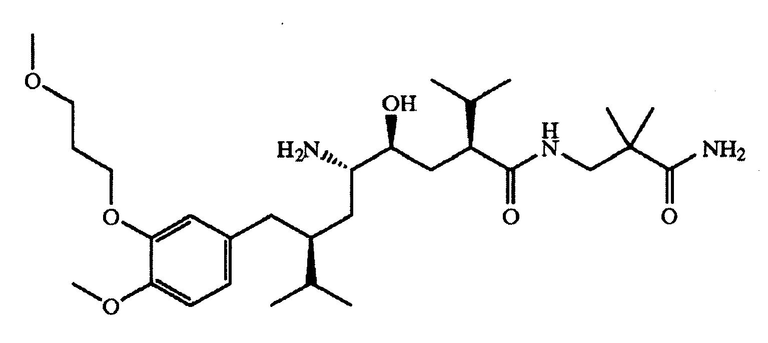

Bekannt ist insbesondere das Hemifumaratsalz von Aliskiren, wie beispielsweise in EP 678 503 in Beispiel 83 offenbart. Das Hemifumarat hat folgende Struktur:

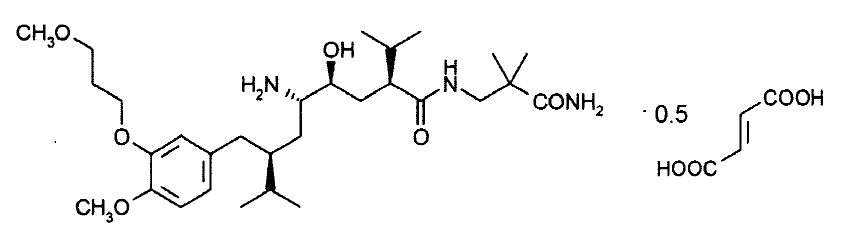

Sofern nichts anderes angegeben ist, schließt der Begriff "Aliskiren" in dieser Anmeldung die freie Base als auch Salze und Solvate davon ein.

Im Stand der Technik sind Darreichungsformen von Aliskiren bekannt, die einen hohen Wirkstoffgehalt aufweisen und den Wirkstoff im Wesentlichen ohne zeitliche Verzögerung freisetzen. WO 2004/002466 A1 beschreibt den Zusatz von Oberflächen-aktiven Mitteln ("surfactants") zur Erhöhung der Bioverfügbarkeit von Aliskiren. Aliskiren ist gemäß dem "Biopharmaceutics Classification System" (BCS) als Klasse III-Wirkstoff einzustufen. Das heißt, der Wirkstoff weist eine schlechte Permeabilität, aber hohe Löslichkeit auf. Der limitierende Faktor für die Bioverfügbarkeit ist daher die Permeationsrate.

WO 2005/089729 A2 und US 2006/0018960 A1 offenbaren schnell auflösende Aliskirentabletten und Aliskirentabletten mit sofortiger Freisetzung, die gekennzeichnet sind durch einen Wirkstoffgehalt von > 46 %, bezogen auf das Gesamtgewicht der Formulierung. Es wird beschrieben, dass Aliskiren-Hemifumarat schlecht verarbeitbar ist, u.a. da es aufgrund seiner Nadelform stark zu interpartikulären Brückenbildungen neigt und in Folge dessen eine schlechte Fließfähigkeit und Verarbeitbarkeit zeigt. Zur Verhinderung von Problemen bezüglich der gleichmäßigen Verteilung des Wirkstoffs in der Arzneiform muss daher mit einem Wirkstoffgehalt > 46 % gearbeitet werden. Weiterhin wird die Verarbeitung durch Nassgranulation beschrieben, die ebenfalls den schlechten Eigenschaften des Wirkstoffs entgegenwirken soll.

Die beiden Druckschriften offenbaren ausdrücklich, dass eine Direktverpressung bzw. Trockenkompaktierung des Wirkstoffs nicht möglich ist aufgrund der hohen Hygroskopizität, der nadelförmigen Partikelstruktur und der schlechten Fließfähigkeit des Wirkstoffs. Entsprechend offenbaren WO 2005/089729 A2 und US 2006/0018960 A1 ein Naßgranulierungsverfahren zur Herstellung von Arzneimitteln, die mehr als 46 % Aliskiren enthalten. Der mehrstufige Prozess der Feuchtgranulierung, Trocknung und anschließenden Verpressung zu Tabletten ist im Regelfall hinsichtlich seiner Durchführbarkeit relativ unproblematisch. So lassen sich im Allgemeinen Feuchtgranulate am sichersten und komplikationslosesten tablettieren, insbesondere auch bei niedrig dosierten Darreichungsformen. Deshalb ist die Feuchtgranulierung normalerweise das Mittel der Wahl, und Feuchtgranulate sind das übliche Zwischenprodukt bei der Herstellung von Tabletten.

Nachteilig bei der Feuchtgranulierung ist jedoch, dass besondere Maschinen zur Granulierung eingesetzt werden müssen, Lösungsmittel zur Herstellung der feuchten Masse erforderlich sind und der Wirkstoff bis zum Abschluss der Trocknung einen längeren Zeitraum der Granulierflüssigkeit ausgesetzt ist. Dies kann zu Stabilitätsproblemen führen. Weiterhin erfordert der sich an die Granulierung anschließende Trocknungsschritt zusätzliche Energie, wodurch der Wirkstoff auch über einen längeren Zeitraum thermischen Einflüssen ausgesetzt wird. Insgesamt ist das Verfahren der Feuchtgranulierung bei einem zum Polymorphismus neigenden und stabilitätslabilen Wirkstoff hinsichtlich des Feuchteeinflusses ungeeignet.

Es besteht daher ein Bedarf an pharmazeutischen Darreichungsformen, welche den Wirkstoff Aliskiren enthalten und Vorteile gegenüber den Darreichungsformen des Standes der Technik aufweisen. Die pharmazeutischen Darreichungsformen sollten insbesondere einfach und ökonomisch herstellbar sein und dennoch gute Eigenschaften bei der Verabreichung aufweisen. Dazu sollte der Wirkstoff möglichst gleichmäßig innerhalb der pharmazeutischen Darreichungsform verteilt sein, auch bei einem Wirkstoffgehalt unter 46 Gew.-%.

Diese Aufgabe wird durch den Gegenstand der Patentansprüche gelöst. Die Erfindung betrifft damit eine pharmazeutische Zusammensetzung, die zur Direktverpressung bzw. Trockenverarbeitung in Tabletten geeignet ist, umfassend den Wirkstoff Aliskiren oder eines seiner pharmazeutisch verträglichen Solvate oder Salze oder ein Solvat eines Salzes in frei fließender Form.

Die Erfindung betrifft ebenfalls die aus diesen pharmazeutischen Zusammensetzungen durch Direktverpressung erhältlichen Tabletten und das Verfahren, mit dem die Zusammensetzungen zu Tabletten verpresst werden.

Es wurde gefunden, dass eine pharmazeutische Zusammensetzung, welche den frei fließenden Wirkstoff Aliskiren enthält, direkt zu Tabletten verpresst werden kann, ohne dass zuvor eine Feuchtgranulierung erforderlich ist, wenn der Wirkstoff vor der Direktverpressung mikronisiert und/oder mit einem pharmazeutisch verträglichen Polymer überzogen wurde. Die Mikronisierung kann mit oder ohne Oberflächenstabilisator durchgeführt werden.

In einer Ausführungsform der Erfindung wird der Wirkstoff somit durch Zerkleinerungstechnologien vor der Direktverpressung bearbeitet. Dadurch wird der Kristallhabitus des Wirkstoffs verändert und die Brückenbildung verhindert. Da Aliskirenfumarat unter mechanischer Einwirkung zur Umwandlung in den amorphen Wirkstoff neigt, der eine geringere Stabilität aufweist, ist bei der Zerkleinerung auf die Temperatur zu achten Die Temperatur sollte während der Zerkleinerung unter 40°C, vorzugsweise unter 35 °C, z. B. bei ca. 10 bis ca. 34 °C oder ca. 20 bis ca 30°C, gehalten werden, um einen Abbau zu verhindern. Es können alle bekannten Verfahren der pharmazeutischen Mikronisierung angewendet werden, beispielsweise unter Verwendung einer Luftstrahlmühle oder Kugelmühle. Bei Verwendung einer Luftstrahlmühle wird vorzugsweise mit einer Prozessluft von 4-6 bar gearbeitet. Eine Verarbeitung unter Schutzgasatmosphäre ist ebenfalls möglich. Die zerkleinerten Kristalle haben vorzugsweise eine mittlere Größe von 3 - 250 µm, vorzugsweise von 5 - 150 µm, am bevorzugtesten von 10 - 120 µm.

Einer möglichen Agglomerierung des mikronisierten Wirkstoffes kann durch Zusatz von einem oder mehreren Oberflächenstabilisatoren engegengewirkt werden. Diese können vor oder während der Zerkleinerung zugesetzt werden. Als Oberflächenstabilisatoren können pharmazeutisch verträgliche organische oder anorganische Hilfstoffe eingesetzt werden, die Polymere, niedermolekulare Oligomere oder nichtionische, ionische, zwitterionische oberflächenaktive Substanzen sind oder diese enthalten. Sie werden in einem Wirkstoff : Hilfsstoff-Gewichtsverhältnis von 1 : 1 bis 1 : 100, bevorzugt von 1 : 2 bis 1 : 50, zugesetzt. Beispiele für Öberflächenstabilisatoren im Sinne der vorliegenden Erfindung sind Hydroxypropylmethylcellulose (HPMC), Casein, Gelatine, Polyvinylpyrrolidon (PVP), Natriumdodecylsulfat (SDS), Traganth, Stearinsäue, Gummi Arabicum, Polaxomer, Polyethylenglykol (PEG), Tween^{®}, Polysaccharide, Alginate, Phospholipide usw.

In einer weiteren Ausführungsform kann der Kristallhabitus und die Oberfläche der Kristalle des Wirkstoffs durch eine Beschichtung derart geändert werden, dass eine Brückenbildung verhindert wird und die Fließfähigkeit und damit die Dosisgenauigkeit erhöht wird. Hierfür können alle gängigen pharmazeutisch verträglichen Polymere eingesetzt werden, beispielsweise Polymere auf Cellulosebasis, Methacrylate oder Polyvinylalkohol (PVA). Beispiele pharmazeutisch verträglicher Polymere sind Celluloseether wie Hydroxypropylcellulose, Hydroxymethylcellulose, Methylcellulose und Hydroxypropylmethylcellulose, Celluloseacetatphthalat, wobei Hydroxypropylmethylcellulose besonders bevorzugt ist. Geeignete Hydroxypropylmethylcellulosen sind beispielsweise unter der Bezeichnung "Methocel" erhältlich. Es wird ferner auf *"*Fiedler - Lexikon der Hilfsstoffe", Editio Cantor-Verlag Aulendorf, 5. Auflage 2002, verwiesen.

Aufgrund der guten Löslichkeitseigenschaften des Aliskirens führt diese Methode auch durch Anlöseerscheinungen an der Oberfläche der Kristalle zu einer Glättung die wiederum vorteilhaft für die weitere Verarbeitung durch Direktverpressung ist. Weiterhin führt der Zusatz solcher Polymere, die auch als Hydrophilisierungsmittel gelten, zur Verbesserung der Bioverfügbarkeit des Wirkstoffs. Die Beschichtung des Wirkstoffs wird vorzugsweise in Wirbelschicht- oder mittels Sprühtrocknung durchgeführt.

Die Polymere werden zur Beschichtung vorzugsweise in einer Menge von 2 - 20 Gew.-%, bevorzugter von 5 - 15 Gew.-%, am bevorzugtesten von 7 - 12 Gew.-%, jeweils bezogen auf das Gewicht des Wirkstoffs, eingesetzt. Die Beschichtungslösung wird auf Grundlage eines organischen Lösungsmittels hergestellt. Geeignete organische Lösungsmittel sind Ethanol oder Isopropanol.

Je nach verwendetem Polymer wird dabei üblicherweise mit einer Produkttemperatur zwischen 30 und 45°C oder zwischen 35 und 40°C gearbeitet.

Ein weiterer Vorteil dieser Verarbeitungsmethode ist die Stabilisierung des Wirkstoffes gegen äußere Einflüsse, wie z.B. Feuchtigkeit, die eine Umwandlung in den thermolabilen amorphen Zustand begünstigt. Dadurch kann das Arzneimittel in kostengünstigeren Packmitteln auf den Markt gebracht werden.

Eine Abwandlung dieser Technologie ist die Einarbeitung des Wirkstoffs in einen Hilfsstoff (vorzugsweise in ein Polymer) und das Aufziehen dieser Lösung auf einen Träger. Hierzu wird der Wirkstoff in einer Lösung eines pharmazeutisch verträglichen Hilfsstoffs (z. B. Polymers) in einem Lösungsmittel gelöst bzw. suspendiert und auf einen inerten Kern aufgezogen, durch bekannte Layeringverfahren wie z.B. die Wirbelschicht. Möglich sind jegliche Art von "multiple units" - Pellets, Minitabletten, Partikel u.s.w.

Für eine schnell freisetzende Formulierung wird vorzugsweise eine Mischung aus einem wasserlöslichen und einem wasserunlöslichen Hilfsstoff verwendet. Es ist aber auch möglich, nur wasserunlösliche oder nur wasserlösliche Hilfsstoffe einzusetzen. Geeignete wasserlösliche Hilfsstoffe sind beispielsweise HPMC, Povidon, Povidon VA 64 (Copolymer des Polyvinylpyrrolidon mit Polyvinylacetat, kommerziell erhältlich von der Firma BASF), Mannitol, Sorbitol. Geeignete wasserunlösliche Hilfsstoffe sind beispielsweise Polyvinylalkohol, Celluloseacetatphthalat, verschiedene Methacrylate etc.

Diese Möglichkeit verbindet sowohl die verbesserte Stabilisierung des Wirkstoffes durch eine innige Einbettung in ein Polymer, als auch die verbesserte Verarbeitbarkeit des Träger-Wirkstoff - Gemisches. Es treten hierdurch keine Brückenbildungen des Wirkstoffes auf, reproduzierbare Herstellung hinsichtlich der gleichmäßigen Verteilung des Wirkstoffs ist sichergestellt.

Entgegen der Darstellung im Patent WO 2005/089729 können durch die Trockenkompaktierung des Wirkstoffes mit oder ohne Hilfsstoffzusatz die Eigenschaften hinsichtlich der Fließfähigkeit und Verarbeitbarkeit verbessert werden. Es können hiermit reproduzierbare Schütt- und Stampfdichten erzeugt werden, die eine volumendosierte Verarbeitung per Tablettierung und damit die "Content Uniformity" sicherstellen. Es zeigte sich, dass der Zusatz eines Bindemittels den Effekt noch verbessern kann bzw. auch einen positiven Einfluß auf die Stabilität ausübt.

Hierzu können alle gängigen Trockenkompaktierverfahren per Walzenkompaktierung oder Brikettkompaktierung eingesetzt werden. Es sollte darauf geachtet werden, dass die Presskraft zwischen 2 - 50 kN, vorzugsweise zwischen 3 und 30 kN, z.B. 5 - 40 kN, liegt.

Im Anschluß kann der Wirkstoff mit den gängigen pharmazeutischen Hilfsstoffen zu einer Tablette direktverpresst werden, die im Anschluß zur Geschmackskaschierung des bitteren Wirkstoffes noch gecoatet werden kann.

Als Bindemittel wird vorzugsweise ein wasserlöslicher Hilfsstoff, z.B. Povidon, HPMC, HPC, eingesetzt. Als wasserlöslicher Hilfsstoff kann jedes pharmazeutisch verträgliche, geschmacksneutrale Material verwendet werden.

Vorzugsweise wird ein wasserlösliches Füllmittel zugesetzt: z. B. Lactose und/oder andere Zuckeralkohole bzw. Kohlenhydrate. Diese können mit einem Anteil an wasserunlöslichen Füllmitteln kombiniert werden, vorzugsweise mit mikrokristalliner Cellulose. Denkbar als Füllmittel sind aber auch anorganische Salze: Calciumcarbonat, Calciumhydrogenphosphat etc.

Um den schnellen Zerfall und die damit korrelierte Freisetzung zu gewährleisten sollte ein Sprengmittel eingesetzt werden. Vorzugsweise Crosspovidon, Natriumcarboxymethylstärke oder ähnliche Superdesintegrants.

Als Schmiermittel können alle üblichen pharmazeutischen Hilfsstoffe eingesetzt werden - vorzugsweise Magnesiumstearat oderLubritab (dehydrit. Planzenöl).

Die Kombination der Hilfsstoffe sollte so gewählt sein, dass der Trockenverlust der Tablettiermischung zwischen 0,2 - 7 %, vorzugsweise zwischen 1,3 - 4% liegt.

Durch die Vorbehandlung des Wirkstoffes zeigen die Fertigmischungen eine gute Reproduzierbarkeit hinsichtlich der Schütt- und Stampfdichte und des Quotienten aus selbigen. Die Schüttdichte liegt vorzugsweise zwischen 0,4 - 0,8 kg/l und der Quotient aus Stampf- und Schüttdichte zwischen 1,1 zu 1,3

Die Bestandteile werden üblicherweise in einem Freifallmischer (Turbula) zusammen gemischt und anschließend auf einer Rundläuferpresse (Fette) zu Tabletten verpresst. Hierbei zeigten die Tablettiermischungen eine gute Kompressibilität. Die Tabletten weisen eine Härte zwischen 60 - 120 N, bevorzugt 70 - 110 N, auf kombiniert mit einer Friabilität kleiner 1 %.

Die vorliegende Erfindung betrifft weiterhin ein Verfahren zur Herstellung eines Arzneimittels, umfassend folgende Schritte:
- Zerkleinern von Aliskiren-Kristallen mit oder ohne Oberflächenstabilisator;
- gegebenenfalls Mischen der zerkleinerten Aliskiren-Kristalle mit einem oder mehreren Hilfsstoffen;
- Direktverpressung der so erhaltenen Zusammensetzung, um eine Tablette zu erhalten, und
- gegebenenfalls Beschichten der so erhaltenen Tablette.

Die Erfindung betrifft weiterhin ein Verfahren zur Herstellung eines Arzneimittels, umfassend folgende Schritte:
- Beschichten von kristallinem Aliskiren mit wenigstens einem pharmazeutisch verträglichen Polymer;
- gegebenenfalls Mischen des beschichteten Aliskirens mit einem oder mehreren Hilfsstoffen;
- Direktverpressung der so erhaltenen Zusammensetzung zu einer Tablette; und
- gegebenenfalls Beschichten der so erhaltenen Tablette

Die Erfindung betrifft weiterhin ein Verfahren zur Herstellung eines Arzneimittels, umfassend folgende Schritte:
- Kompaktieren von kristallinem Aliskiren mit wenigstens einem pharmazeutisch verträglichen Hilfsstoff;
- gegebenenfalls Mischen des kompaktierten Aliskirens mit einem oder mehreren Hilfsstoffen;
- Direktverpressung der so erhaltenen Zusammensetzung zu einer Tablette; und
- gegebenenfalls Beschichten der so erhaltenen Tablette.

In bevorzugten Ausführungsformen der erfindungsgemäßen Verfahren wird keine Naßgranulierung oder Feuchtgranulierung durchgeführt.

Weiterhin betrifft die Erfindung die Verwendung der oben beschriebenen pharmazeutischen Zusammensetzung oder Tablette zur Behandlung von Bluthochdruck oder einer damit assoziierten Erkrankung. Zu diesen Erkrankungen zählen unter anderem kongestives Herzversagen, Cardiohypertonie, Cardiofibrose, Postinfarktcardiomyopathie, Komplikationen in Folge von Diabetes, wie Nephropathie, Vaskolopathie und Neuropathie, coronare Gefäßerkrankungen, Restenose nach einer Angioplastie, erhöhter Intraokulardruck, Glaukom, abnormales Vaskularwachstum, Hyperaldosteronismus, Angstzustände und Kognitionsstörungen.

Die Erfindung betrifft weiterhin die Verwendung von Aliskiren zur Herstellung eines Arzneimittels zur Behandlung von Bluthochdruck oder einer damit assoziierten Erkrankung, **dadurch gekennzeichnet, dass** eine Aliskiren-enthaltende pharmazeutische Zusammensetzung zu Tabletten direktverpresst wird. Vorzugsweise wird der Wirkstoff Aliskiren vor der Direktverpressung durch wenigstens ein pharmazeutisch verträgliches Polymer beschichtet. Alternativ können die Aliskiren-Kristalle vor der Direktverpressung zerkleinert werden, beispielsweise durch Mikronisierung wie oben beschrieben.

Erfindungsgemäß ist es.ebenfalls möglich, dass die hierin beschriebenen pharmazeutischen Zusammensetzungen und Tabletten in einer Kombinationstherapie verabreicht werden. Dabei können die erfindungsgemäßen Arzneimittel gleichzeitig mit einem weiteren Wirkstoff verabreicht werden oder in zeitlichem Abstand. Als weitere Wirkstoffe kommen beispielsweise folgende Wirkstoffkategorien in Betracht: AT₁-Rezeptorantagonisten, ACE-Inhibitoren, Betablocker, Calciumkanalblocker, Aldosteronsynthaseinhibitoren, Aldosteronrezeptorantagonisten, und Diuretika. Beispiele dieser Wirkstoffe sind dem Fachmann wohlbekannt.

Die Erfindung wird durch folgende Beispiele weiter veranschaulicht:

### Beispiel 1

Mengenangaben werden berechnet auf die Einzeldosis verwendet.

Der Wirkstoff wurde mit 300 mg Calciumphosphat, 5 mg Aerosil, 40 mg Crosscarmellose und 20 mg Magnesiumstearat im Kompaktor bei 15 - 45 kN kompaktiert. Das Kompaktat wurde mit einem Sieb zerkleinert und mit den Restmengen vom Calciumphosphat, Magnesiumstearat und Aerosil auf einem Freifallmischer aufgemischt. Diese Fertigmischung wurde auf einer Rundläuferpresse zu Tabletten verpresst. Diese wiesen eine Härte von 70 - 110 N auf verbunden mit einer Friabilität kleiner 1%. Anschließend wurden die Tabletten in einem Trommelcoater gecoatet. Hierfür wurde eine Suspension aus HPMC, PEG, Talkum, Titandioxid und dem Farbstoff hergestellt.

### Trockenkompaktierung:

| **Nr.** | **Inhaltsstoff** | **Produkt** | **Funktion** | **[mg]** | **% pro D.F.** |
|---|---|---|---|---|---|
| 1 | Aliskiren | Hemifumarat | Wirkstoff | 331,5* | 37,7 |
| 2 | Calciumphosphat | Di-Cafos AN | Verdünnungsmittel | 412,5 | 46,9 |
| 3 | Kolloidales Siliziumdioxid | Aerosil | Gleitmittel | 10,0 | 1,1 |
| 4 | Croscarmellose Sodium | Ac-Di Sol | Sprengmittel | 40,0 | 4,5 |
| 5 | Magnesiumstearat | Stearate Mg | Schmiermittel | 45,0 | 5,1 |
| 6 | Hydroxypropyl Methylcellulose | Methocel E5 premium LV | Filmbildner | 30,0 | 3,4 |
| 7 | Polyethylenglycol | Polyglykol 8000P | Weichmacher | 4,5 | 0,5 |
| 8 | Talc | Talc,micron. | Antihaftmittel | 3,5 | 0,4 |
| 9 | Titandioxid | Titandioxid | Färbemittel | 2,5 | 0,3 |
| 10 | Färbemittel | Nicht bestimmt | Färbemittel | 0,5 | 0,1 |
| | **Gesamt** | | | 880,0 | 100,0 |

| | | | | | |
|---|---|---|---|---|---|
| *entspricht 300 mg Aliskiren-Base | | | | | |

### Beispiel 2

Der Wirkstoff wurde in einem Wirbelschichtgerät (Glatt GPC3) vorgelegt und mit einer isopropanolischen Lösung von HPMC gecoatet. Die Produkttemperatur lag zwischen 35-40°C bei anliegender Zulufttemperatur von 40 -80 °C. Der Sprühdruck wurde mit 1-2 bar eingestellt. Anschließend wurde der gecoatete Wirkstoff mit Avicel, Aerosil und Crosscarmellose für 30 min auf einem Freifallmischer vorgemischt. Die Endmischung wurde mit Zugabe von Magnesiumstearat hergestellt. Hierzu wurde nochmals für 3 min aufgemischt. Die Fertigmischung wurde anschließend auf einer Rundläuferpresse zu Tabletten verpresst (Fette 102i) mit einer Härte von 60 -120 N.

Bei Bedarf kann die Tablette äquivalent zu Beispiel 1 gecoatet werden.

### Wirkstoff-Coating:

| **Nr.** | **Inhaltsstoff** | **Produkt** | **Funktion** | **[mg]** | **% pro D.F.** |
|---|---|---|---|---|---|
| 1 | Aliskiren | Hemifumarat | Wirkstoff | 331,5* | 39,0 |
| 2 | Hydroxypropyl Methylcellulose | Methocel E5 | Überzugs-Filmbildner | 30,0 | 3,5 |
| 3 | Microcrystalline Cellulose | Avicel | Verdünnungsmittel | 436,0 | 51,3 |
| 4 | Kolloidales Siliziumdioxid | Aerosil | Gleitmittel | 2,5 | 0,3 |
| 5 | Croscarmellose Sodium | Ac-Di Sol | Sprengmittel | 35,0 | 4,1 |
| 6 | Magnesiumstearat | Stearate Mg | Schmiermittel | 15,0 | 1,8 |
| | **Gesamt** | | | 850,0 | 100,0 |

| | | | | | |
|---|---|---|---|---|---|
| *entspricht 300 mg Aliskiren-Base | | | | | |

### Beispiel 3

Der Wirkstoff wurde zusammen mit Gummi arabicum in der Luftstrahlmühle zerkleinert. Es wurde eine Prozessluft zwischen 4 -6 bar angelegt. Nach der Vermahlung wurde das Wirkstoff-Hilfsstoffgemisch mit Aerosil, Crospovidon und Avicel für 30 min auf einem Freifallmischer aufgemischt. Anschließend wurde nochmals mit Magnesiumstearat für 3 min gemischt. Die Fertigmischung wurde analog zu den vorherigen Beispiele verpresst und ggf. gecoatet.

### Wirkstoff-Zerkleinerung:

| **Nr.** | **Inhaltsstoff** | **Produkt** | **Funktion** | **[mg]** | **% pro D.F.** |
|---|---|---|---|---|---|
| 1 | Aliskiren | Hemifumarat | Wirkstoff | 331,5* | 37,6 |
| 2 | Gummi Arabicum | Arabisch Gummi | Oberflächenstabilisator | 331,5 | 37,6 |
| 3 | Microcrystalline Cellulose | Avicel | Verdünnungsmittel | 120,0 | 13,7 |
| 4 | Kolloidales Siliziumdioxid | Aerosil | Gleitmittel | 8,0 | 0,9 |
| 5 | quervernetztes Povidon | Crospovidon | Sprengmittel | 80,0 | 9,0 |
| 6 | Magnesiumstearat | Stearate Mg | Schmiermittel | 10,0 | 1,2 |
| 7 | **Gesamt** | | | 881,0 | 100,0 |

| | | | | | |
|---|---|---|---|---|---|
| *entspricht 300 mg Aliskiren-Base | | | | | |

## Patentansprüche

1. Pharmazeutische Zusammensetzung, die zur Direktverpressung von Tabletten geeignet ist, umfassend den Wirkstoff Aliskiren oder eines seiner pharmazeutisch verträglichen Solvate oder Salze in frei fließender Form.

2. Pharmazeutische Zusammensetzung nach Anspruch 1, **dadurch gekennzeichnet, dass** der Wirkstoff in mikronisierter Form vorliegt.

3. Pharmazeutische Zusammensetzung nach Anspruch 1 oder 2, **dadurch gekennzeichnet, dass** der Wirkstoff durch ein pharmazeutisch verträgliches Polymer beschichtet ist.

4. Pharmazeutische Zusammensetzung nach Anspruch 3, **dadurch gekennzeichnet, dass** das pharmazeutisch verträgliche Polymer ausgewählt ist aus der Gruppe bestehend aus Polymeren auf Cellulosebasis, Methacrylaten und Polyvinylalkohol.

5. Pharmazeutische Zusammensetzung nach Anspruch 3 oder 4, **dadurch gekennzeichnet, dass** der Gehalt des pharmazeutisch verträglichen Polymers 2 - 20 Gew.-% bezogen auf das Gewicht des Wirkstoffs beträgt.

6. Pharmazeutische Zusammensetzung nach einem der vorhergehenden Ansprüche, **dadurch gekennzeichnet, dass** sie ein Bindemittel enthält.

7. Pharmazeutische Zusammensetzung nach einem der vorhergehenden Patentansprüche, **dadurch gekennzeichnet, dass** die Schüttdichte der Zusammensetzung zwischen 0,4 und 0,7 kg/L liegt.

8. Pharmazeutische Zusammensetzung nach einem der vorhergehenden Ansprüche, **dadurch gekennzeichnet, dass** der Quotient aus Stampfdichte und Schüttdichte zwischen 1,1 und 1,3 liegt.

9. Verfahren zur Herstellung einer Tablette, umfassend die Direktverpressung einer pharmazeutischen Zusammensetzung nach einem der vorhergehenden Ansprüche.

10. Verfahren nach Anspruch 9, **dadurch gekennzeichnet, dass** die Presskraft während der Direktverpressung zwischen 2 und 50 kN liegt.

11. Tablette erhältlich durch ein Verfahren nach Anspruch 9 oder 10.

12. Tablette enthaltend mikronisiertes oder durch ein pharmazeutisch verträgliches Polymer beschichtetes Aliskiren.

13. Verwendung einer pharmazeutischen Zusammensetzung nach einem der Ansprüche 1 - 8 oder einer Tablette nach Anspruch 11 oder 12 zur Herstellung eines Medikaments zur Behandlung von Bluthochdruck oder einer damit assoziierten Erkrankung.

14. Verwendung nach Anspruch 13, **dadurch gekennzeichnet, dass** die mit Bluthochdruck assoziierte Erkrankung ausgewählt ist aus der Gruppe bestehend aus kongestivem Herzversagen, Cardiohypertonie, Cardiofibrose, Postinfarktcardiomyopathie, Komplikationen in Folge von Diabetes, wie Nephropathie, Vaskolopathie und Neuropathie, coronare Gefäßerkrankungen, Restenose nach einer Angioplastie, erhöhtem Intraokulardruck, Glaukom, abnormalem Vaskularwachstum, Hyperaldosteronismus, Angstzuständen und Kognitionsstörungen.

15. Verwendung nach Anspruch 13 oder 14, **dadurch gekennzeichnet, dass** die pharmazeutische Zusammensetzung oder Tablette in Kombination mit einem weiteren Wirkstoff verabreicht wird.

16. Verwendung nach Anspruch 15, **dadurch gekennzeichnet, dass** der weitere Wirkstoff ausgewählt ist aus der Gruppe bestehend aus AT₁-Rezeptorantagonisten, ACE-Inhibitoren, Betablockern, Calciumkanalblockern, Aldosteronsynthaseinhibitoren, Aldosteronrezeptorantagonisten, und Diuretika.
